# EUROPÄISCHE PATENTSCHRIFT

(11) **EP 0 984 901 B1**
(45) Veröffentlichungstag und Bekanntmachung des Hinweises auf die Patenterteilung: **30.07.2003**
(21) Anmeldenummer: 98934899.0
(22) Anmeldetag: 26.05.1998
(51) Int. Cl.: C01B 25/32, C04B 35/447, A61K 9/20, A61L 27/00

(54) **HYDROXYLAPATIT-GEL**
HYDROXYLAPATITE GEL
GEL D'HYDROXYLAPATITE

(30) Priorität: 30.05.1997 BG 10154497
(43) Veröffentlichungstag der Anmeldung: 15.03.2000
(73) Patentinhaber: Ewers, Rolf, Prof. Dr. Dr., A-1160 Wien (AT); Spassova, Else, 1180 Wein (AT)
(72) Erfinder: EWERS, Rolf, A-1180 Wien (AT); SPASSOVA, Else, A-1180 Wien (AT); JORDANOVA, Margarita Nikolova, 1404 Sofia (BG); DJEROV, Dimitar Assenov, 1000 Sofia (BG); DRAMOV, Sava Assenov, 1172 Sofia (BG); KIROV, Gueorgui Nikolov, Produene-Sofia (BG); VELICHKOVA, Velitchka Alexandrova, 1309 Sofia (BG); TCHAKALSKI, Kiril Ranguelov, 1614 Sofia (BG); ANDREEV, Andrey Ivanov, 1000 Sofia (BG); IVANOV, Emil Stoimenov, 1330 Sofia (BG); BAUMANN, Arnulf-Univ. Klinik für Mund-, Kiefer AKH, 1090 Wein (AT)
(74) Vertreter: Kleinschmidt, Michael, Dr.
(86) Internationale Anmeldenummer: EP9803093
(87) Internationale Veröffentlichungsnummer: WO98054089

(56) Entgegenhaltungen:
- EP-A- 0 211 676
- EP-A- 0 284 074
- WO-A-87/03491
- DE-A- 4 130 546
- SADA E. ET AL.: "Hydrothermal synthesis of crystalline hydroxyapatite ultrafine particles" CHEMICAL ENGINEERING COMMUNICATIONS, Bd. 103, 1991, Seiten 57-64, XP002079971
- KOJI IOKU ET AL.: "Dense/porous layered apatite ceramics prepared by HIP post-sintering" JOURNAL OF MATERIALS SCIENCE LETTERS., Bd. 8, Nr. 10, 10. Oktober 1989, Seiten 1203-1204, XP002079710 LONDON GB
- CHEMICAL ABSTRACTS, vol. 121, no. 6, 8. August 1994 Columbus, Ohio, US; abstract no. 63482, MIN K. S. ET AL: "Synthesis of ultra-fine hydroxyapatite powders by hydrothermal reaction" XP002079713 & YOOP HAKHOECHI, Bd. 29, Nr. 12, 1992, Seiten 997-1003,
- CHEMICAL ABSTRACTS, vol. 122, no. 10, 6. März 1995 Columbus, Ohio, US; abstract no. 112770, CHOI J. W. ET AL.: "Sintering behavior of ultra-fine hydroxyapatite powders synthesized by hydrothermal reaction" XP002079712 & YOOP HAKHOECHI , Bd. 31, Nr. 11, 1994, Seiten 1265-1270,
- HAYEK ET AL: "Pentacalcium monohydroxyorthophosphate" INORGANIC SYNTHESIS, Bd. 7, 1963, Seiten 63-67, XP002080600
- CHEMICAL ABSTRACTS, vol. 113, no. 6, 6. August 1990 Columbus, Ohio, US; abstract no. 46344, NONAMI TORU ET AL.: "Manufacture of bone substitute with porous hydroxylapatite and tricalcium phosphate" XP002080841 & JP 01 293877 A (TDK CORP.) 27. November 1989
- CHEMICAL ABSTRACTS, vol. 122, no. 20, 15. Mai 1995 Columbus, Ohio, US; abstract no. 248429, ISHIZAWA HITOSHI: "A method for manufacture of implants coated with calcium phosphate" XP002080862 & JP 07 047116 A (NIPPON KOGAKU KK) 21. Februar 1995
- DATABASE WPI Section Ch, Week 9215 Derwent Publications Ltd., London, GB; Class A96, AN 92-120276 XP002080865 & JP 04 064362 A (INOUE K) , 28. Februar 1992

## Beschreibung

Die Erfindung betrifft eine formbare Hydroxylapatit-Masse, dieses enthaltende Formkörper sowie deren Verwendung, insbesondere im Bereich der Knochenchirurgie.

Im Bereich der Knochenchirurgie besteht ein Bedarf an Knochenersatzstoffen und Implantaten, welche körperverträglich und gut zu bearbeiten sind. Um das Anwachsen im Körper zu erleichtern, sollte das Knochenimplantat eine Struktur aufweisen, die dem der Knochen möglichst ähnlich ist. Außerdem ist eine hohe mechanische Stabilität wünschenswert. Das Implantatmaterial sollte zudem als Träger für Wirkstoffe wie beispielsweise wachstumsfördernde oder -hemmende Substanzen geeignet sein.

Als Knochenersatzmaterial hat sich ein Hydroxylapatit-Material bewährt, welches aus dem Calciumcarbonat-Skelett von kalkinkrustierenden Algen gewonnen wird. Ein derartiges Material ist in der DE 37 09 897 C2 beschrieben. Zur Herstellung von Formkörpern wird das durch hydrothermale Synthese erhaltene granuläre Hydroxylapatit-Material mit gelöschtem Kalk als Bindemittel in einen Formkörper eingerüttelt und danach nochmals einer hydrothermalen Behandlung unterzogen. Das so erhaltene Knochenimplantat besitzt eine hohe interkonnektive Porosität und eine hohe spezifische Oberfläche. In seinem Chemismus und kristallinen Aufbau ist es den Knochen wesentlich ähnlicher als andere Knochenersatzmaterialien.
In einigen speziellen Anwendungen sind jedoch Knochenersatzmaterialien erforderlich, die eine noch höhere Porosität und besonders hohe mechanische Stabilität aufweisen.

In der Veröffentlichung von Sada et al "Hydrothermal synthesis of crystalline hydroxylapatite ultrafine particles" in Chemical Engineering Communications, Bd. 103, 1991, Seiten 57 bis 64 wird die Herstellung von ultrafeinem Hydroxylapatitpulver beschrieben, in dem eine alkalische wäßrige Lösung je eines Calcium- und Phosphatsalzes in einem molaren Verhältnis von Ca:P von 1,67 zu einem nanokristallinen, gelartigem Hydroxylapatit umgesetzt wird, welches dann durch eine hydrothermale Behandlung in nanokristallines Hydroxylapatitpulver umgewandelt wird.

In der Veröffentlichung von Nonami et al. "Manufacture of bone substitue with porous hydroxylapatite and tricalcium phosphate", chemical abstracts, vol. 113, no.6, abstract no. 46344 und JP-A-01 293877 von TDK Corp. wird das Vermischen von porösem Hydroxylapatit und Calciumphosphat in einem Gewichtsverhältnis von 10:1 bis 1:10 beschrieben, wobei die Mischung anschließend unter hohem Druck und Wärmeeinwirkung in einen Formförper umgewandelt wird.

**Aufgab**e der Erfindung ist es, Materialien anzugeben, welche sich zur Herstellung eines Knochenersatzmaterials eignen, welches in seinem Chemismus und dem kristallinen Aufbau natürlichen Knochen möglichst ähnlich ist, eine sehr poröse Mikrostruktur aufweist, dabei aber eine hohe mechanische Stabilität besitzt. Die Materialien sollten einfach herstellbar, kostengünstig und gut verarbeitbar sein. Zusätzlich sollten sie es erlauben, bei ihrer Verarbeitung den Kristallphasen-Anteil und die Festigkeit des Knochenersatzmaterials gezielt einzustellen.

Die Lösung dieser Aufgabe gelingt der formbaren Hydroxylapatit-Masse gemäß Anspruch 1 und der verdichteten Hydroxylapatit-Masse gemäß Anspruch 14, welche wiederum Bestandteil des erfindungsgemäßen Hydroxylapatit-Formkörpers gemäß Anspruch 15 sind. Die Erfindung betrifft weiterhin die Verwendung des Hydroxylapatit-Formkörpers gemäß Anspruch 16. Der Hydroxylapatit-Formkörper ist ebenfalls als Beschichtung in einem Metallimplantat gemäß Anspruch 19 vorhanden. Außerdem betrifft die Erfindung ein Verfahren zur Herstellung des Metallimplantats gemäß Anspruch 20, sowie ein Verfahren zur Herstellung des Hydroxylapatit-Formkörpers gemäß Anspruch 22.
Weitere Verfahrensvarianten und Ausbildungsformen ergeben sich aus den Unteransprüchen.

In einem ersten Aspekt betrifft die Erfindung eine formbare Hydroxylapatit-Masse, welche durch einen Sol-Gel-Prozeß erhältlich ist. Dabei wird eine alkalische wässrige Lösung eines Calciumsalzes mit einer alkalischen wässrigen Lösung eines Phosphatsalzes zunächst'zu einem Sol umgesetzt. Die Stöchiometrie wird dabei derart gewählt, daß das Verhältnis von Calcium zu Phosphor dem Verhältnis im Hydroxylapatit entspricht. Das molare Verhältnis von Calcium zu Phosphor bei der Sol-Herstellung liegt also im Bereich von etwa 1,67. Anschließend wird das so erhaltene Sol durch hydrothermale Behandlung in ein Gel umgewandelt.

Der pH-Wert bei der Sol-Herstellung liegt vorzugsweise in einem Bereich von 9 bis 12 und besonders zweckmäßig zwischen 10,5 und 11. Der gewünschte pH-Bereich kann durch Zugabe der üblichen Basen eingestellt werden. Besonders geeignet hierfür ist Ammoniak. Es hat sich zudem als vorteilhaft herausgestellt, zunächst beide wässrigen Lösungen getrennt auf den gewünschten pH-Wert einzustellen und den pH-Wert, falls erforderlich, nach dem Mischen beider Lösungen nochmals in den gewünschten Bereich zu bringen.

Vorteilhafterweise erfolgt die Sol-Herstellung in einem Temperaturbereich zwischen 10° und 40°C, besonders zweckmäßig bei Raumtemperatur (20° bis 25°C).

Bei der Herstellung des Sols können beispielsweise 0,1 bis 1N wässrige Lösungen des Calciumsalzes bzw. des Phosphatsalzes verwendet werden. Besonders geeignet sind 0,3 bis 0,5N wässrige Lösungen der Salze.
Als Ausgangsmaterialien können alle löslichen Calcium- und Phosphatsalze eingesetzt werden, die möglichst keine körperunverträglichen Bestandteile enthalten sollten. Ein geeignetes Calciumsalz ist beispielsweise Calciumnitrat. Als Phosphatsalz kann Diammoniumhydrogenphosphat verwendet werden.

Unter den genannten Bedingungen bildet sich das Sol nach dem Mischen der beiden wässrigen Lösungen durch Stehenlassen für einen Zeitraum von mehreren Tagen. In einem zweiten Schritt wird das Sol dann durch hydrothermale Behandlung in ein Gel umgewandelt. Die hydrothermale Behandlung erfolgt zweckmäßig in einem Temperaturbereich von 180 bis 200°C. Besonders vorteilhaft wird sie in einem Autoklaven durchgeführt. Geeignet sind insbesondere mit Polytetrafluorethylen oder ähnlichen inerten Auskleidungen beschichtete Autoklaven. Von Vorteil ist es, den Autoklaven maximal zu zwei Dritteln zu füllen und die hydrothermale Behandlung unter dem sich dann einstellenden Sättigungsdampfdruck der Lösung durchzuführen. Der pH-Wert bei der Umsetzung zum Gel liegt vorteilhafterweise im selben Bereich wie bei der Sol-Herstellung, also zwischen 9 und 12 und insbesondere zwischen 10,5 und 11.

Während der hydrothermalen Behandlung bilden sich in Sol Kristallkeime und feine Kristalle des Hydroxylapatits. Je nach Dauer der hydrothermalen Behandlung des Sols können die Kristallphasen-Anteile im Hydroxylapatit-Gel gezielt gesteuert werden. Auf diese Weise können die physikalischen Eigenschaften des Hydroxylapatit-Gels gezielt beeinflußt werden. Vorteilhaft erfolgt die hydrothermale Behandlung solange, bis der Anteil an Kristallkeimen und feinkristallinem Hydroxylapatit im Gel über 80 % und insbesondere etwa 90 % beträgt. In der Gelmasse bleiben bis zu zwei Drittel des im Sol enthaltenen Wassers erhalten. Üblicherweise enthält das erfindungsgemäße Gel nach seiner Herstellung bis zu 70 Gew.-% Wasser. Ein mittlerer Wassergehalt liegt bei etwa 60 Gew.-%. Läßt man das Hydroxylapatit-Gel längere Zeit stehen, setzt sich das Gel ab und es bildet sich ein wäßriger Überstand. Durch Abgießen dieses Überstandes läßt sich der Wassergehalt des Gels, falls gewünscht, gezielt verringern.

Diese formbare Hydroxylapatit-Masse nach Anspruch 1, umfaßt neben dem oben beschriebenen Hydroxylapatit-Gel, welches als Bindemittel fungiert, einen calcium-haltigen granulären Füllstoff. Als Füllstoffe geeignet sind beispielsweise alle solchen, welche im Bereich der Knochenchirurgie bereits eingesetzt werden. Besonders geeignet als granulärer Füllstoff sind Hydroxylapatit-Materialien, welche aus kalkinkrustierenden Algen gewonnen werden. Beispiele sind im deutschen Patent 37 09 897 beschrieben. In den dort beschriebenen Materialien bleibt das Calciumcarbonat-Skelett der Algen erhalten, so daß das Material eine hohe interkonnektive Porosität und eine große spezifische Oberfläche aufweist.

Als granulärer Füllstoff für die formbare Hydroxylapatit-Masse kann auch ein modifiziertes, Tricalciumphosphat-haltiges Hydroxylapatit-Material verwendet werden, das erhältlich ist durch Umsetzen eines von organischen Verbindungen befreiten Algen-Hartgewebes in einer alkalischen wässrigen Phosphat-Lösung unter Zusatz von Mg²⁺-Ionen bei erhöhter Temperatur. Ein derartiges Material ist in einer parallelen deutschen Patentanmeldung der Anmelderin beschrieben. Dieses Tricalciumphosphathaltige Hydroxylapatit-Material weist vorteilhaft einen Tricalciumphosphat-Gehalt von 20 bis 90 Gew.% auf. Das Algen-Hartgewebe wird, wie im Fall der nichtmodifizierten Hydroxylapatit-Materialien, zweckmäßig aus kalkinkrustierenden Meeresalgen gewonnen, insbesondere aus solchen der Spezies Corallinacea oder Codiacea.

In der formbaren Hydroxylapatit-Masse können das erfindungsgemäße Hydroxylapatit-Gel und der granuläre Feststoff in einem breiten Mengenverhältnis miteinander gemischt werden. Geeignet sind beispielsweise Gewichtsverhältnisse von Hydroxylapatit-Gel und granulärem Feststoff zwischen 10:1 und 1:10, wobei hier von einem Hydroxylapatit-Gel mit einem Wassergehalt von etwa 60 Gew.-% ausgegangen wird. Die jeweiligen Anteile werden entsprechend der gewünschten späteren Verwendung der erfindungsgemäßen formbaren Hydroxylapatit-Masse gewählt. Auch die Art und Weise, auf welche die formbare Masse verarbeitet werden soll, spielt bei der Auswahl der Anteile der beiden Komponenten eine Rolle. Bei der Verwendung der erfindungsgemäßen formbaren Hydroxylapatit-Masse für eine Beschichtung können höhere Anteile an Gel die Verarbeitung erleichtern. Formkörper allein aus Hydroxylapatit-Masse - beispielsweise Knochenersatzmaterialien, welche eine hohe mechanische Festigkeit besitzen müssen - werden üblicherweise einen höheren Anteil an granulärem Feststoff enthalten. Für letztere sind beispielsweise Gewichtsverhältnisse von Gel zu Feststoff zwischen 1:5 und 1:8 verwendbar.

Die erfindungsgemäße formbare Hydroxylapatit-Masse kann weitere Komponenten enthalten, wie sie beispielsweise bei Knochenersatzmaterialien oder Filtermaterialien üblich sind. Für Knochenersatzmaterial kann es beispielsweise zweckmäßig sein, der Masse wenigstens einen Wirkstoff zuzusetzen. Beispiele sind wachstumsfördernde oder -hemmende Substanzen. Spezielle Beispiele sind Antibiotika, Chemotherapeutika, tumorhemmende Verbindungen und knocheninduktive Substanzen. Beispielhaft für letztere können knochenmorphogene Proteine (bone morphogenic proteins) genannt werden. Diese Wirkstoffe können in die erfindungsgemäße Masse eingearbeitet werden, oder sie werden auf den fertigen, aus der erfindungsgemäßen Masse hergestellten Formkörper aufgetragen. Zweckmäßig wird für jeden Wirkstoff die jeweils klinisch aktive Menge verwendet.

Die erfindungsgemäße formbare Hydroxylapatit-Masse ist durch ihren Anteil an Hydroxylapatit-Gel beliebig formbar. Dies erleichtert erheblich die Herstellung auch kompliziert aufgebauter Formkörper. Wenn als granulärer Füllstoff ein aus kalkinkrustierenden Algen gewonnenes Hydroxylapatit-Material verwendet wird, wird eine formbare Masse erhalten, die praktisch ausschließlich aus Hydroxylapatit besteht. Die aus dieser Masse hergestellten Formkörper sind nicht nur außerordentlich körperverträglich, sondern sie besitzen auch eine außergewbhnlich poröse Mikrostruktur bei gleichzeitig hoher mechanischer Stabilität. Die erfindungsgemäße Hydroxylapatit-Masse ist deshalb außerordentlich gut zur Herstellung von Knochenersatzmaterialien geeignet. Zudem lassen sich die physikalischen Eigenschaften der formbaren Masse und infolgedessen auch der aus ihr hergestellten Formkörper gezielt beeinflussen.

Beispielsweise ist es möglich, die Hydroxylapatit-Masse unter Druck zu komprimieren und damit ihre Dichte und ihre Festigkeit zu erhöhen. Wendet man zum Komprimieren einen Druck an, der 1 MPa nicht übersteigt, bleibt die poröse Mikrostruktur der Masse dabei erhalten.

Auf die Steuerung der Kristallphasen-Anteile im Gel - und damit auch in der das Gel enthaltenden formbaren Hydroxylapatit-Masse - war bereits hingewiesen worden. Die Kristallinität kann weiterhin über die Art und Dauer der thermischen Behandlung der formbaren Hydroxylapatit-Masse beeinflußt werden, mit der aus der Masse ein fester Formkörper erzeugt wird.

Zu diesem Zweck wird die erfindungsgemäße formbare Hydroxylapatit-Masse in eine geeignete Form gefüllt und vor dem Aushärten zweckmäßig zunächst entgast. Geeignet ist hierzu beispielsweise eine Behandlung durch Ultraschall. Um eine größere Dichte und höhere Festigkeit zu erreichen, kann die formbare Masse, wie erwähnt, komprimiert werden. Die thermische Behandlung zum Aushärten der Masse erfolgt zweckmäßig in einem Temperaturbereich zwischen 500° und 650°C, insbesondere zwischen 550° und 600°C. Die Dauer der thermischen Behandlung richtet sich nach der Zusammensetzung der formbaren Masse und der Größe des Formkörpers. Üblicherweise wird die Zeit der Behandlung zwischen etwa einer halben Stunde und mehreren Stunden betragen. Falls erforderlich, kann der hergestellte erfindungsgemäße Formkörper mit üblichen OP-Werkzeugen nachbearbeitet und in seine endgültige Form gebracht werden. Außerdem können Beschichtungen, wie solche aus knocheninduktiven Verbindungen, die bereits erwähnt wurden, auf den erfindungsgemäßen Hydroxylapatit-Formkörper aufgebracht werden.

Wegen der sehr porösen Mikrostruktur bei hoher mechanischer Festigkeit eignet sich der erfindungsgemäße Hydroxylapatit-Formkörper ausgezeichnet zur Verwendung im Bereich der Knochenchirurgie, insbesondere als Knochenersatzmaterial oder als Trägermaterial für Wirkstoffe. Wegen ihrer porösen Struktur können die erfindungsgemäßen Formkörper jedoch auch als Filtermaterialien eingesetzt werden.

Die erfindungsgemäßen Hydroxylapatit-Formkörper eignen sich außerdem als bioaktive Beschichtung auf Metallimplantaten und insbesondere auf Titanknochenimplantaten. Die Erfindung betrifft in einem weiteren Aspekt ein derartiges Metallimplantat, welches eine Beschichtung aus dem erfindungsgemäßen Hydroxylapatit-Formkörper umfaßt. Zur Herstellung des Implantats wird auf die Metalloberfläche eine Schicht aus der erfindungsgemäßen formbaren Hydroxylapatit-Masse aufgebracht und diese einer thermischen Behandlung zwischen 500° und 650°C unterzogen, wie dies vorstehend beschrieben wurde. Um die Haftung der Beschichtung auf der Metalloberfläche zu verbessern, wird diese Oberfläche zweckmäßig vor ihrer Beschichtung behandelt. Derartige Behandlungen zur Haftungsverbesserung sind für Metallimplantate bekannt. Diese bekannten Oberflächenbehandlungsverfahren können auch vor Aufbringung der erfindungsgemäßen Beschichtung angewendet werden. Bevorzugt wird jedoch eine Behandlung durchgeführt, die ebenfalls Gegenstand dieser Erfindung ist.

Im bevorzugten erfindungsgemäßen Verfahren zur Herstellung eines Metallimplantats wird die Oberfläche des Metalls vor Aufbringung der Beschichtung in einer Elektrolytlösung unter Funkenentladung bei einer Temperatur zwischen -10 und -20°C oxidiert, bis die Dicke der Oxidschicht zwischen 25 und 40 µm beträgt. Die Oxidation erfolgt bei Wechselstrom von 50 Hz bei einer Spannung zwischen 110 und 200 V. Die verwendete wäßrige Elektrolytlösung umfaßt:

| | |
|---|---|
| Polyethylenglykol (Molekulargewicht 200 bis 400) | 80 bis 200 ml/l |
| | |
| Wenigstens eine Chlorsauerstoffsäure oder deren Salz | 5 bis 20 g/l |
| | |
| sowie | |
| | |
| Amin | 10 bis 30 ml/l |
| und/oder | |
| | |
| Flußsäure oder deren Salze | 2 bis 25 g/l |
| und/oder | |
| | |
| Phosphorsäure oder deren Salze | 20 bis 80 g/l |
| und/oder | |
| | |
| Perborsäure oder deren Salze | 10 bis 40 g/l |

Als Oxidationsmittel werden bevorzugt die Alkalisalze der Chlorsauerstoffsäuren verwendet, hier inbesondere die Chlorate und Chlorite wie Natriumchlorat oder Natriumchlorit. Es können auch Gemische verschiedener Salze verwendet werden. Von den übrigen Säuren werden vorzugsweise die Alkali- oder Ammoniumsalze verwendet. Beispiele bevorzugter Verbindungen sind Natriumfluorid, Ammoniumfluorid, Kaliumdihydrogenphosphat und Natriumperborat.
Als Amin werden zweckmäßig aliphatische Amine oder Alkohol-Amine eingesetzt, bevorzugt Triethylamin oder Triethanolamin.

Die gemäß dem erfindungsgemäßen Verfahren erhaltene Oxidschicht wird anschließend in einem Brennprozeß rekristallisiert. Zweckmäßig wird die Oxidschicht nach dem Trocknen für 20 bis 40 Minuten einer Temperatur von 550 bis 650°C ausgesetzt. Von Vorteil werden die eventuell noch auf der Oxidschicht befindlichen ungebundenen Ionen mit destilliertem Wasser entfernt, bevor die erfindungsgemäße formbare Masse auf die behandelte Metalloberfläche aufgetragen wird. Zweckmäßig beträgt die Schichtdicke 5 bis 10 µm. Die thermische Behandlung der formbaren Masse erfolgt wie vorstehend beschrieben.

Die Erfindung soll nachfolgend anhand einiger Beispiele näher erläutert werden.

### Herstellung des Hydroxylapatit-Gels

Eine 0,5N wässrige Lösung von Ca(NO₃)₂·4H₂O und eine 0,3N wäßrige Lösung von (NH₄)₂HPO₄ werden getrennt voneinander mit Ammoniak auf einen pH-Wert von 10,5 eingestellt. Beide Lösungen werden im Verhältnis 1:1 unter Rühren gründlich gemischt. Der pH-Wert der so erhaltenen Lösung wird, falls erforderlich, erneut mit Ammoniak auf 10,5 gebracht.
Die Reaktionsmischung wird bei einer Temperatur zwischen 20° und 25°C fünf bis sechs Tage stehengelassen. Während dieser Zeit bildet sich ein primäres Apatit-Sol.

Zur Herstellung eines Gels wird das Sol bei 180 bis 200°C hydrothermal behandelt. Hierzu wird das Gel in einen mit PTFE ausgekleideten Autoklaven überführt, der maximal zu zwei Dritteln befüllt wird. Die hydrothermale Behandlung erfolgt unter dem Sättigungsdampfdruck der Lösung. Die Behandlung wird solange fortgesetzt, bis sich wenigstens 80% Kristallkeime und feinkristalliner Hydroxylapatit gebildet haben. Vorzugsweise wird die hydrothermale Behandlung fortgesetzt, bis sich etwa 90% Kristallkeime und feinkristalliner Hydroxylapatit gebildet haben. Die hydrothermale Behandlung erfordert üblicherweise wenigstens 24 Stunden. Nach etwa 30 Stunden Reaktionszeit ist keine wesentliche Änderung der Kristallinität mehr zu beobachten. Das erhaltene Hydroxylapatit-Gel besitzt einen Wasseranteil von etwa 60 Gew.-%.

### Herstellung einer formbaren Hydroxylapatit-Masse

Das erhaltene Hydroxylapatit-Gel wird mit einem gemäß deutschem Patent 37 09 897 aus kalkinkrustierenden Rotalgen hergestellten granulären Hydroxylapatit-Material in einem Gewichtsverhältnis von 1:10 gemischt. Es wird eine homogene teigartige Masse erhalten.

### Herstellung eines Hydroxylapatit-Formkörpers

Die erhaltene Hydroxylapatit-Masse wird in eine Preßform gefüllt. Sie wird durch Bestrahlung mit Ultraschall entlüftet und bei einem Druck von 0,9 MPa einige Minuten lang verdichtet. Anschließend wird die verdichtete Masse in der Preßform in einem Ofen bei 550 bis 600°C etwa 60 Minuten lang thermisch behandelt. Auf diese Weise wird ein Formkörper mit einer sehr porösen Mikrostruktur und hoher mechanischer Festigkeit erhalten. Er läßt sich mechanisch nachbearbeiten.

### Herstellung eines Hydroxylapatit-beschichteten Metallimplantats

### 1. Vorbehandlung des Metallimplantats

Ein Titan-Knochenimplantat wird unter Funkenentladung in einer Elektrolytlösung bei -10°C oxidiert. Die Lösung enthält:

| | |
|---|---|
| Polyethylenglykol (MG 200) | 100 ml/l |
| Natriumchlorat/chlorit | 5 g/l |
| Natriumfluorid | 21 g/l |
| Kaliumdihydrogenphosphat | 68 g/l |
| Triethylamin | 10 ml/l |

Die Oxidation wird bei einer Spannung von 160 V 5 Minuten lang durchgeführt. Dann wird das behandelte Implantat aus der Lösung entfernt, und die erzeugte Oxidschicht wird bei 600°C in einem Ofen zwanzig Minuten lang rekristallisiert. Anschließend wird mehrfach mit kochendem destillierten Wasser gespült, bis alle nicht gebundenen Ionen völlig vom Implantat entfernt sind.

### 2. Vorbehandlung des Metallimplantats (2. Variante)

Die Oberfläche des Titan-Knochenimplantats wird unter Funkenentladung in einer Elektrolytlösung bei -15°C oxidiert. Die Lösung besteht aus:

| | |
|---|---|
| Polyethylenglykol (MG 400) | 150 ml/l |
| Natriumchlorat/chlorit | 10 g/l |
| Natriumfluorid | 21 g/l |
| Kaliumdihydrogenphosphat | 68 g/l |
| Natriumperborat | 38 g/l |
| Ammoniumfluorid | 6 g/l |
| Triethanolamin | 15 ml/l |

Die Durchführung des Verfahrens erfolgt wie unter Punkt 1, jedoch bei einer Spannung von 180 V 3,5 Minuten lang. Analog Punkt 1 erfolgt die Rekristallisation der Oxidschicht bei 580°C für 30 Minuten. Die Nachbehandlung entspricht ebenfalls Punkt 1.

### 3. Aufbringen der Beschichtung

Das gemäß Punkt 1 oder 2 behandelte Metallimplantat wird mit einer Beschichtung versehen, die besteht aus einem Hydroxylapatit-Gel wie oben beschrieben und einem gemäß deutschem Patent 37 09 897 hergestellten Hydroxylapatit-Granulat in einem Volumenverhältnis von 2:0,75. Die Schichtdicke beträgt etwa 7 µm. Nach dem Trocknen der Schicht wird diese bei 550°C in einem Ofen 40 Minuten lang fixiert.

### 4. Aufbringen der Beschichtung (Variante 2)

Es wird wie unter Punkt 3 beschrieben vorgegangen, jedoch beträgt das Verhältnis von Hydroxylapatit-Gel zu Granulat 2:1. Die Fixierung der Schicht erfolgt 30 Minuten lang bei 500°C.

In beiden Fällen wird eine Beschichtung mit außerordentlich guten bioaktiven Eigenschaften erhalten. Die Beschichtung ist gut mit der Metalloberfläche verbunden. Das erfindungsgemäße beschichtete Metallimplantat ergab in klinischen Untersuchungen äußerst günstige Bedingungen für eine Knochenaugmentation und eine gute Befestigung des neu gebildeten Knochengewebes an der Oberfläche.

## Patentansprüche

1. Formbare Hydroxylapatit-Masse,
**dadurch gekennzeichnet,**
**daß** sie einen calciumhaltigen granulären Füllstoff und ein Hydroxylapatit-Gel umfaßt, welches erhältlich ist durch einen Sol-Gel-Prozeß, in dem eine alkalische wässrige Lösung eines Calciumsalzes mit einer alkalischen wässrigen Lösung eines Phosphatsalzes mit einem molaren Verhältnis von Calcium zu Phosphor im Bereich von 1,67 zu einem Sol umgesetzt werden und das Sol durch hydrothermale Behandlung in ein Gel umgewandelt wird.

2. Formbare Hydroxylapatit-Masse gemäß Anspruch 1,
**dadurch gekennzeichnet,**
**daß** der pH-Wert bei der Sol-Herstellung in einem Bereich von 9 bis 12 und insbesondere von 10,5 bis 11 liegt.

3. Formbare Hydroxylapatit-Masse gemäß Anspruch 1 oder 2,
**dadurch gekennzeichnet,**
**daß** die Sol-Herstellung bei 10°C bis 40°C und insbesondere bei 20°C bis 25°C erfolgt.

4. Formbare Hydroxylapatit-Masse gemäß einem der Ansprüche 1 bis 3,
**dadurch gekennzeichnet,**
**daß** 0,1 bis 1N wässrige Lösungen des Calciumsalzes und des Phosphatsalzes und insbesondere 0,3 bis 0,5N wässrige Lösungen eingesetzt werden.

5. Formbare Hydroxylapatit-Masse gemäß einem der Ansprüche 1 bis 4,
**dadurch gekennzeichnet,**
**daß** als Calciumsalz Calciumnitrat eingesetzt wird.

6. Formbare Hydroxylapatit-Masse gemäß einem der Ansprüche 1 bis 5,
**dadurch gekennzeichnet,**
**daß** als Phosphatsalz Diammoniumhydrogenphosphat eingesetzt wird.

7. Formbare Hydroxylapatit-Masse gemäß einem der Ansprüche 1 bis 6,
**dadurch gekennzeichnet,**
**daß** die hydrothermale Behandlung bei 180°C bis 200°C erfolgt.

8. Formbare Hydroxylapatit-Masse gemäß Anspruch 7,
**dadurch gekennzeichnet,**
**daß** die hydrothermale Behandlung solange erfolgt, bis der Anteil an Kristallkeimen und feinkristallinem Hydroxylapatit über 80 % und insbesondere etwa 90 % beträgt.

9. Formbare Hydroxylapatit-Masse gemäß einem der Ansprüche 1 bis 8,
**dadurch gekennzeichnet,**
**daß** als granulärer Füllstoff ein aus kalkinkrustierenden Algen gewonnenes Hydroxylapatit-Material dient.

10. Formbare Hydroxylapatit-Masse gemäß Anspruch 9,
**dadurch gekennzeichnet,**
**daß** als granulärer Füllstoff ein Tricalciumphosphat-haltiges Hydroxylapatit-Material dient, welches erhältlich ist durch Umsetzen eines von organischen Verbindungen befreiten Algen-Hartgewebes in einer alkalischen wässrigen Phosphat-Lösung unter Zusatz von Mg²⁺-Ionen bei erhöhter Temperatur.

11. Formbare Hydroxylapatit-Masse gemäß Anspruch 10,
**dadurch gekennzeichnet,**
**daß** der Tricalciumphosphat-Gehalt 20 bis 90 Gew.%, bezogen auf den granulären Füllstoff, beträgt.

12. Formbare Hydroxylapatit-Masse gemäß einem der Ansprüche 8 bis 11,
**dadurch gekennzeichnet,**
**daß** Hydroxylapatit-Gel und granulärer Füllstoff in einem Gewichts-Verhältnis von 10:1 bis 1:10 und insbesondere von 1:5 bis 1:8 vorhanden sind.

13. Formbare Hydroxylapatit-Masse gemäß einem der Ansprüche 1 bis 12,
**gekennzeichnet durch** einen Gehalt an wenigstens einem Wirkstoff, insbesondere einer wachstumsfördernden oder wachstumshemmenden Verbindung, einem Antibiotikum, einem Chemotherapeutikum, einer tumorhemmenden Verbindung oder einer knocheninduktiven Verbindung, insbesondere wenigstens einem knochenmorphogenen Protein.

14. Verdichtete Hydroxylapatit-Masse, erhältlich durch Komprimieren der formbaren Hydroxylapatit-Masse gemäß einem der Ansprüche 1 bis 13 bei einem Druck von bis zu 1 MPa.

15. Hydroxylapatit-Formkörper, erhältlich durch thermische Behandlung der Hydroxylapatit-Masse gemäß einem der Ansprüche 1 bis 14 bei einer Temperatur von 500°C bis 650°C und insbesondere von 550°C bis 600°C.

16. Verwendung des Hydroxylapatit-Formkörpers gemäß Anspruch 15 in der Knochenchirurgie, insbesondere als Knochenersatzmaterial oder als Trägermaterial für Wirkstoffe, und als Filtermaterial.

17. Verwendung des Hydroxylapatit-Formkörpers gemäß Anspruch 16 als Trägermaterial für wachstumsfördernde oder wachstumshemmende Verbindungen, ein Antibiotikum, ein Chemotherapeutikum, eine tumorhemmende Verbindung oder eine knocheninduktive Verbindung, insbesondere wenigstens ein knochenmorphogenes Protein.

18. Verwendung gemäß Anspruch 16 oder 17 als bioaktive Beschichtung auf Metallimplantaten und insbesondere auf Titan-Knochenimplantaten.

19. Metallimplantat, insbesondere Titan-Knochenimplantat,
**dadurch gekennzeichnet,**
**daß** es eine Beschichtung aus dem Hydroxylapatit-Formkörper gemäß Anspruch 15 umfaßt.

20. Verfahren zur Herstellung eines Metallimplantats, insbesondere eines Titan-Knochenimplantats, gemäß Anspruch 19,
**dadurch gekennzeichnet,**
**daß** auf die Oberfläche des Metallimplantats eine Schicht aus der formbaren Hydroxylapatit-Masse gemäß einem der Ansprüche 1 bis 13 aufgebracht und diese einer thermischen Behandlung bei einer Temperatur zwischen 500°C und 650°C unterzogen wird.

21. Verfahren gemäß Anspruch 20,
**dadurch gekennzeichnet,**
**daß** die Oberfläche des Metallimplantats vor Aufbringung der Beschichtung in einer Elektrolytlösung unter Funkenentladung bei einer Temperatur zwischen -10°C und -20°C oxidiert wird, bis die Dicke der Oxidschicht zwischen 25 und 40 µm beträgt, wobei die Oxidation bei Wechselstrom von 50 Hz bei einer Spannung 110 V bis 200 V in einer Elektrolytlösung erfolgt, welche umfaßt:
| | |
|---|---|
| Polyethylenglykol (Molekulargewicht 200 bis 400) | 80 bis 200 ml/l |
| wenigstens eine Chlorsauerstoffsäure oder deren Salz | 5 bis 20 g/l |
| sowie | |
| Amin | 10 bis 30 ml/l |
| und/oder | |
| Flußsäure oder deren Salze | 2 bis 25 g/l |
| und/oder | |
| Phosphorsäure oder deren Salze | 20 bis 80 g/l |
| und/oder | |
| Perborsäure oder deren Salze | 10 bis 40 g/l. |

22. Verfahren zur Herstellung eines Hydroxylapatit-Formkörpers gemäß Anspruch 15,
**dadurch gekennzeichnet,**
**daß** calciumhaltiger granulärer Füllstoff und Hydroxylapatitgel miteinander vermischt und einer thermischen Behandlung bei einer Temperatur von 500°C bis 650°C und insbesondere von 550°C bis 600°C unterzogen werden.

23. Verfahren gemäß Anspruch 22,
**dadurch gekennzeichnet,**
**daß** die Hydroxylapatit-Masse vor der thermischen Behandlung entgast und insbesondere mittels Ultraschall-Bestrahlung entgast wird.

24. Verfahren gemäß Anspruch 22 oder 23,
**dadurch gekennzeichnet,**
**daß** die Hydroxylapatit-Masse vor der thermischen Behandlung komprimiert wird.

25. Verfahren gemäß Anspruch 24,
**dadurch gekennzeichnet,**
**daß** der Formkörper in einer Preßform mit einem Druck von bis zu 1 MPa behandelt wird.

## Claims

1. A moldable hydroxylapatite composition, which comprises a calcium-containing granular filler and a hydroxylapatite gel which is obtainable by a sol-gel process in which an alkaline aqueous solution of a calcium salt is reacted with an alkaline aqueous solution of a phosphate salt with a molar ratio of calcium to phosphorus in the region of 1.67 to give a sol, and the sol is converted by hydrothermal treatment into a gel.

2. A moldable hydroxylapatite composition as claimed in claim 1, wherein the pH during the production of the sol is in a range from 9 to 12 and, in particular, from 10.5 to 11.

3. A moldable hydroxylapatite composition as claimed in claim 1 or 2, wherein the sol is produced at from 10°C to 40°C and, in particular, at from 20°C to 25°C.

4. A moldable hydroxylapatite composition as claimed in any of claims 1 to 3, wherein 0.1 to 1 N aqueous solutions of the calcium salt and of the phosphate salt and, in particular, 0.3 to 0.5 N aqueous solutions are employed.

5. A moldable hydroxylapatite composition as claimed in any of claims 1 to 4, wherein calcium nitrate is employed as calcium salt.

6. A moldable hydroxylapatite composition as claimed in any of claims 1 to 5, wherein diammonium hydrogen phosphate is employed as phosphate salt.

7. A moldable hydroxylapatite composition as claimed in any of claims 1 to 6, wherein the hydrothermal treatment takes place at from 180°C to 200°C.

8. A moldable hydroxylapatite composition as claimed in claim 7, wherein the hydrothermal treatment takes place until the proportion of crystal nuclei and microcrystalline hydroxylapatite is above 80% and, in particular, about 90%.

9. A moldable hydroxylapatite composition as claimed in any of claims 1 to 8, wherein a hydroxylapatite material obtained from calcareous algae is used as granular filler.

10. A moldable hydroxylapatite composition as claimed in claim 9, wherein a tricalcium phosphate-containing hydroxylapatite material which is obtainable by reacting an algal hard tissue, from which organic compounds have been removed, in an alkaline aqueous phosphate solution with addition of Mg²⁺ ions at elevated temperature is used as granular filler.

11. A moldable hydroxylapatite composition as claimed in claim 10, wherein the tricalcium phosphate content is from 20 to 90% by weight based on the granular filler.

12. A moldable hydroxylapatite composition as claimed in any of claims 8 to 11, wherein hydroxylapatite gel and granular filler are present in a ratio of from 10:1 to 1:10 and, in particular, from 1:5 to 1:8 by weight.

13. A moldable hydroxylapatite composition as claimed in any of claims 1 to 12, which comprises at least one substance, in particular a growth-promoting or growth-inhibiting compound, an antibiotic, a chemotherapeutic agent, a tumor-inhibiting compound or a bone-inductive compound, in particular at least one bone morphogenic protein.

14. A compacted hydroxylapatite composition obtainable by compression of the moldable hydroxylapatite composition as claimed in any of claims 1 to 13 under a pressure of up to 1 MPa.

15. A hydroxylapatite molding obtainable by thermal treatment of the hydroxylapatite composition as claimed in any of claims 1 to 14 at a temperature of from 500°C to 650°C and, in particular, from 550°C to 600°C.

16. The use of the hydroxylapatite molding as claimed in claim 15 in bone surgery, in particular as bone substitute material or as carrier material for active substances, and as filter material.

17. The use of the hydroxylapatite molding as claimed in claim 16 as carrier material for growth-promoting or growth-inhibiting compounds, an antibiotic, a chemotherapeutic agent, a tumor-inhibiting compound or a bone-inductive compound, in particular at least one bone morphogenic protein.

18. The use as claimed in claim 16 or 17 as bioactive coating on metal implants and, in particular, on titanium bone implants.

19. A metal implant, in particular titanium bone implant, which comprises a coating of the hydroxylapatite molding as claimed in claim 15.

20. A process for producing a metal implant, in particular a titanium bone implant, as claimed in claim 19, which comprises applying to the surface of the metal implant a layer of the moldable hydroxylapatite composition as claimed in any of claims 1 to 13, and subjecting the latter to a thermal treatment at a temperature between 500°C and 650°C.

21. The process as claimed in claim 20, wherein the surface of the metal implant is, before application of the coating, oxidized in an electrolyte solution with spark discharge at a temperature between -10°C and -20°C until the thickness of the oxide layer is between 25 and 40 µm, the oxidation taking place with a 50 Hz alternating current and with a voltage of 110 V to 200 V in an electrolyte solution which comprises:
| | |
|---|---|
| polyethylene glycol (molecular weight 200 to 400) | 80 to 200 ml/l |
| at least one chlorine oxoacid or salt thereof | 5 to 20 g/l |
| and | |
| amine | 10 to 30 ml/l |
| and/or | |
| hydrofluoric acid or salts thereof | 2 to 25 g/l |
| and/or | |
| phosphoric acid or salts thereof | 20 to 80 g/l |
| and/or | |
| perboric acid or salts thereof | 10 to 40 g/l |

22. A process for producing a hydroxylapatite molding as claimed in claim 15, which comprises a calcium-containing granular filler and hydroxylapatite gel being mixed together and subjected to a thermal treatment at a temperature of from 500°C to 650°C and, in particular, of from 550°C to 600°C.

23. The process as claimed in claim 22, wherein the hydroxylapatite composition is degassed before the thermal treatment and is, in particular, degassed by exposure to ultrasound.

24. The process as claimed in claim 22 or 23, wherein the hydroxylapatite composition is compressed before the thermal treatment.

25. The process as claimed in claim 24, wherein the molding is treated in a compression mold under a pressure of up to 1 MPa.

## Revendications

1. Pâte d'hydroxylapatite pouvant être modelée, **caractérisée en ce qu'**elle contient une matière de remplissage granuleuse à base de calcium et un gel d'hydroxylapatite lequel peut être obtenu par un procédé sol-gel dans lequel une solution aqueuse alcaline d'un sel de calcium est transformée en un sol avec une solution aqueuse d'un sel de phosphate avec un rapport molaire du calcium par rapport au phosphore situé dans le domaine de 1,67, et le sol est transformé en gel par un traitement hydrothermal.

2. Pâte d'hydroxylapatite pouvant être modelée selon revendication 1, **caractérisée en ce que** la valeur du pH se situe lors de la fabrication du sol dans un domaine compris entre 9 et 12 et en particulier entre 10,5 et 11.

3. Pâte d'hydroxylapatite pouvant être modelée selon la revendication 1 ou 2, **caractérisée en ce que** la fabrication du sol est réalisée à une température comprise entre 10 °C et 40 °C et en particulier entre 20 °C et 25 °C.

4. Pâte d'hydroxylapatite pouvant être modelée selon l'une des revendications 1 à 3, **caractérisée en ce qu**'on utilise des solutions aqueuses du sel de calcium et du sel de phosphate comprises entre 0,1 et 1 N et en particulier des solutions aqueuses comprises entre 0,3 et 0,5 N.

5. Pâte d'hydroxylapatite pouvant être modelée selon l'une des revendications 1 à 4, **caractérisée en ce que** du nitrate de calcium est utilisé comme sel de calcium.

6. Pâte d'hydroxylapatite pouvant être modelée selon l'une des revendications 1 à 5, **caractérisée en ce que** du di-ammonium d'hydrogenphosphate est employé comme sel de phosphate.

7. Pâte d'hydroxylapatite pouvant être modelée selon l'une des revendications 1 à 6, **caractérisée en ce que** le traitement hydrothermal est réalisé à une température comprise entre180 °C et 200 °C.

8. Pâte d'hydroxylapatite pouvant être modelée selon la revendication 7, **caractérisée en ce que** le traitement hydrothermal dure jusqu'à ce que la teneur en germes cristallins et en fins cristaux d'hydroxylapatite soit supérieure à 80% et en particulier s'élève à environ 90%.

9. Pâte d'hydroxylapatite pouvant être modelée selon l'une des revendications 1 à 8, **caractérisée en ce qu**'on utilise comme matière de remplissage granuleuse un matériau d'hydroxylapatite obtenu à partir d'algues incrustant du calcaire.

10. Pâte d'hydroxylapatite pouvant être modelée la selon la revendication 9, **caractérisée en ce qu**'un matériau d'hydroxylapatite contenant du phosphate tricalcique sert de matière de remplissage granuleuse, lequel matériau d'hydroxylapatite peut être obtenu en transformant un tissu dur d'algues débarrassé des composés organiques dans une solution aqueuse alcaline de phosphate sous ajout d'ions Mg²⁺ à haute température.

11. Pâte d'hydroxylapatite pouvant être modelée selon la revendication 10, **caractérisée en ce que** la teneur en phosphate tricalcique est comprise entre 20 et 90 % en poids rapporté à la matière de remplissage granuleuse.

12. Pâte d'hydroxylapatite pouvant être modelée selon l'une des revendications 8 à 11, **caractérisée en ce que** le gel d'hydroxylapatite et la matière de remplissage granuleuse sont présents dans un rapport pondéral compris entre 10:1 et 1:10 et en particulier entre 1:5 et 1:8.

13. Pâte d'hydroxylapatite pouvant être modelée selon l'une des revendications 1 à 12, **caractérisée par** une teneur en au moins un composant actif, en particulier un composant favorisant la croissance ou inhibant la croissance, un antibiotique, un agent chimiothérapeutique, un agent anti-tumeur ou un composant ostéo-inducteur, en particulier au moins une protéine morphogénique de l'os.

14. Pâte d'hydroxylapatite compactée, pouvant être obtenue en comprimant la pâte d'hydroxylapatite pouvant être modelée selon l'une des revendications 1 à 13 à une pression pouvant s'élever jusqu'à 1 MPa.

15. Corps moulé d'hydroxylapatite, pouvant être obtenu par un traitement thermique de la pâte d'hydroxylapatite selon l'une des revendications 1 à 14 à une température comprise entre 500 °C et 650 °C et en particulier entre 550 °C et 600 °C.

16. Utilisation du corps moulé d'hydroxylapatite la selon revendication 15 dans la chirurgie osseuse, en particulier comme matériau de prothèse osseuse ou comme support pour des agents actifs, et comme matériau de filtrage.

17. Utilisation du corps moulé d'hydroxylapatite selon la revendication 16 comme support pour des composants favorisant la croissance ou inhibant la croissance, un antibiotique, un agent chimiothérapeutique, un agent anti-tumeur ou un ostéo-inducteur, en particulier au moins une protéine morphogénique de l'os.

18. Utilisation selon la revendication 16 ou 17 comme revêtement bioactif sur des implants en métal et en particulier sur les implants osseux en titane.

19. Implant en métal, en particulier implant osseux en titane, **caractérisé en ce qu**'il comprend une couche du corps moulé d'hydroxylapatite selon la revendication 15.

20. Procédé pour la fabrication d'un implant en métal, notamment un implant osseux en titane, selon la revendication 19, **caractérisé en ce qu**'une couche de la pâte d'hydroxylapatite pouvant être modelée selon l'une des revendications 1 à 13 est appliquée sur la surface de l'implant en métal et cette couche est soumise à un traitement thermique à une température comprise entre 500 °C et 650 °C.

21. Procédé selon la revendication 20, **caractérisé en ce que** la surface de l'implant en métal est oxydée, avant l'application de la couche, dans une solution d'électrolyte en appliquant des décharges d'arcs à une température comprise entre -10 °C et -20 °C jusqu'à ce que l'épaisseur de la couche d'oxyde soit comprise entre 25 et 40 µm, l'oxydation étant réalisée sous un courant alternatif de 50 hertz avec une tension comprise entre 110 V et 200 V dans une solution d'électrolyte contenant :
| | |
|---|---|
| du glycol de polyéthylène ( poids moléculaire compris entre 200 et 400) | 80 à 200 ml/l |
| au moins un acide du chlore contenant de l'oxygène ou un sel de celui-ci | 5 à 20 grammes /1 |
| ainsi que | |
| une amine | 10 à 30:m1 / 1 |
| et/ou | |
| de l'acide fluorhydrique ou des sels de celui-ci | 2 à 25 g/l |
| et/ou | |
| de l'acide phosphorique ou des sels de celui-ci | 20 à 80 grammes /1 |
| et/ou | |
| de l'acide perborique ou des sels de celui-ci | 10 à 40 g/l. |

22. Procédé de fabrication d'un corps moulé d'hydroxylapatite selon la revendication 15, **caractérisé en ce que** la matière de remplissage granuleuse contenant du calcium et le gel d'hydroxylapatite sont mélangés ensemble et sont soumis à un traitement thermique à une température comprise entre 500 °C et 650 °C et en particulier entre 550 °C et 600 °C.

23. Procédé selon la revendication 22, **caractérisé en ce que** la pâte d'hydroxylapatite est dégazée avant le traitement thermique et en particulier qu'elle est dégazée au moyen d'un traitement par ultrasons.

24. Procédé selon la revendication 22 ou 23, **caractérisé en ce que** la pâte d'hydroxylapatite est comprimée avant le traitement thermique.

25. Procédé selon la revendication 24, **caractérisé en ce que** le corps moulé est traité dans un moule de pressage avec une pression pouvant atteindre 1 MPa.
